# EUROPEAN PATENT SPECIFICATION

(11) **EP 3 629 337 B1**
(45) Date of publication and mention of the grant of the patent: **17.06.2026**
(21) Application number: 18197524.4
(22) Date of filing: 28.09.2018
(51) Int. Cl.: G16H 40/63, G16H 50/50, G16H 30/40, A61C 7/00, G06T 15/50

(54) **RENDERING OF DENTAL MODELS**
RENDERING VON ZAHNMODELLEN
RENDU DE MODÈLES DENTAIRES

(43) Date of publication of application: 01.04.2020
(73) Proprietor: Ivoclar Vivadent AG, 9494 Schaan (LI)
(72) Inventor: Lancelle, Marcel, 8049 Zürich (CH); Mörzinger, Roland, 8050 Zürich (CH); Degen, Nicolas, 8703 Erlenbach (CH); Sörös, Gabor, 8041 Zürich (CH); Nemanja, Bartolovic, 8046 Zürich (CH)
(74) Representative: Uexküll & Stolberg

(56) References cited:
- US-A1- 2013 218 530
- US-A1- 2017 109 931
- US-A1- 2018 153 659
- US-A1- 2018 168 781
- KAPANU AG: "Kapanu-Augmented Reality for the Future of Dentistry", YOUTUBE, 6 March 2017 (2017-03-06), pages 1, XP054979194, Retrieved from the Internet <URL:https://www.youtube.com/watch?v=cIEXAlN_QF4> [retrieved on 20190307]
- ALEKSANDR AMIRKHANOV ET AL: "WithTeeth: Denture Preview in Augmented Reality", 1 January 2018 (2018-01-01), XP055574940, ISBN: 978-3-03868-072-7, Retrieved from the Internet <URL:https://www.cg.tuwien.ac.at/research/publications/2018/amirkhanov-2018-withteeth/amirkhanov-2018-withteeth-paper.pdf> [retrieved on 20190327], DOI: 10.2312/VMV.20181250

## Description

The present invention pertains to a computer-implemented method for visualization of three-dimensional models of a denture or other dental prosthesis (dental model) in an image or image stream of a person's face. In particular, the described method allows visualization of dental models in dental virtual mock-up applications, such as dental augmented reality applications.

In the context of the present invention, the term "denture" is not necessarily restricted to full dentures but also comprises partial dentures, orthodontic situations or adaptations, or dental restorations such as dental prostheses, including crowns, crown lays, veneers, inlays and onlays, bridges, dental implants, and implant restorations. Accordingly, the term "dental model" includes all models of dental prostheses - such as models of complete and partial dentures - that are used for prosthodontic purposes. The dental model may also comprise appliances that are attached to dentures or real teeth, such as dental braces and retainers or mouthguards for several purposes.

For dentists and patients, it is of interest to get a visual impression of the appearance of the patient's face with a modified dental situation, i.e. to visualize the modified dental situation in an image of the face of the patient. Also, the appearance during or after a dental treatment may be of importance for the patient before deciding to undergo such treatment. For this purpose, a virtual preview (virtual mock-up) of the dentition modified by dental treatment is helpful for the dentist and may also be used in the course of interactively modifying the treatment plan to get the most favourable aesthetic results.

In dental virtual mock-up applications, virtual teeth are shown in a photo or video of a person's face or part thereof. Realistic visualization is important in this field, as humans are very sensitive to deviations from reality in human faces. This is a well-known problem called the "uncanny valley".

To overcome the "uncanny valley" problem in a visualization of virtual teeth in a photo or video of a face, realistic lighting of the three-dimensional (3D) rendered content is crucial. Thus, the lighting of the rendered virtual content should match the lighting of the real scene - i.e. essentially the face. So far, no such lighting exists for the application in rendering 3D virtual dentition in dentistry.

In computer game rendering of virtual characters, the character's 3D face geometry is used for generating lighting and shadowing effects. Images or videos of a face however are two-dimensional (2D) and thus do not provide a 3D face geometry.

Another typical solution for this problem includes ray tracing. This enables realistic rendering by taking many physical effects into account. However, this approach is usually too slow for real time applications.

The Article "WithTeeth: Denture Preview in Augmented Reality" by Aleksandr Amirkhanov et al., 1 January 2018 (2018-01-01), XP055574940, DOI: 10.2312/VMV.20181250, ISBN: 978-3-03868-072-7 describes the visualization of a virtual dental model in a two-dimensional image of a face, wherein the contour of the lips in the image of the face is determined and the rendered image of the virtual dental model is cut out along the line of the lips so that only the remaining part visible within the inner mouth region of the image of the face is placed in the region of the inner mouth region in the image of the face. The preamble of claim 1 is based on this Article.

In the YouTube video "Augmented Reality for the future of dentistry" of Kapanu AG, published in March 2017, visualizations of virtual dental models in images of the face of a patient including an inner mouth region are shown. In this visualization certain approximations of the effects of shadowing in the oral cavity in the inner mouth region were included, namely a) using a darker illumination level in a region of the dental model located further back in the oral cavity, and b) including a two-dimensional shadow of the lip line. These are very rough approximations that cannot convincingly visualize the real effects of shadowing.

US 2018/0168781 A1 describes an Augmented Reality (AR) display that is controlled by a computing device to display an image of a patient including an inner mouth region, wherein in the inner mouth region in the image of the AR Display a visualized image of a dental model can be shown. Also described is a light enhancement module that can improve the visibility of image regions within an oral cavity to improve visibility for a dental practitioner. Effects of shadowing of visualized virtual dental models in the oral cavity are not mentioned.

It is therefore an object of the present invention to provide a computer-implemented method that allows realistic rendering and illumination of a virtual three-dimensional dental model, particularly being a representation of a full or partial denture.

It is another object to provide such a method, wherein the resulting rendering of the virtual dental model fits into to a camera image of a face.

It is another object to provide such a method that allows overcoming the uncanny valley problem.

It is another object to provide such a method that allows realistic rendering and illumination of the model in real-time.

It is another object to provide such a method that allows realistic rendering and illumination of the model in augmented-reality (AR) applications.

It is another object to provide such a method that can be performed on a mobile device with limited computing power, particularly in real time and/or with a live video feed.

It is another object to provide such a method that is performed fully or semi-automatically.

It is a further object to provide a handheld mobile device for performing such a method.

At least one of these objects is achieved by the method of claim 1, the mobile device of claim 14 and/or one of the dependent claims of the present application.

A first aspect of the invention relates to a method for realistic visualization of a virtual dental model in a two-dimensional image of a face, the image comprising at least an inner mouth region of the face. The dental model comprises a three-dimensional representation of a dental prosthesis (or part thereof). For instance, the model can be that of a full or partial denture. In particular, the dental model is a digital three-dimensional model of a denture comprising one or more teeth, in particular a plurality of teeth, e. g. six or more teeth, with or without gum (gingiva).

The method comprises estimating or assuming a lighting situation in the image of the face and determining boundaries of the inner mouth region in the image, particularly wherein the boundaries are defined by inner boundaries of lips of the face. The lighting situation comprises one or more virtual point light light sources, in particular also a position of the light sources, image based or global illumination, spherical harmonics or directional lights.

Based on the boundaries of the inner mouth region, on a three-dimensional face geometry and on the lighting situation, a shadowing in the inner mouth region (or spatially: in the region behind the inner mouth region) is taken into account when computing a lighting of the dental model, wherein this computation of the lighting taking into account shadowing is performed by
- generating, for a multitude of surface points (e. g. vertices of the virtual dental model) on the surface of the virtual dental model, a first ray to each of a plurality of virtual point light sources,
- generating for each first ray a second ray to a virtual camera, using an intersection of the first ray with the three-dimensional geometry as a starting point for the second ray, and
- using an intersection of the second ray with an image plane as a lookup location to determine a lighting for the respective surface point, wherein a surface point is assumed to be lighted by a virtual point light source if the second ray intersects the image plane at the inner mouth region.

Finally, the 3D dental model and the 2D image are visualized together, the visualization of the dental model (e. g. a 2D representation of the dental model) having the computed lighting. In particular, at least the inner mouth region in the image is overlaid with the visualization of the dental model.

According to one embodiment of the method, the three-dimensional face geometry is a geometry of the face, i.e. the same face that is shown in the image, wherein the face geometry is acquired using a depth sensor. In particular, the three-dimensional face geometry can be acquired together with the two-dimensional image or as a range image.

According to an alternative embodiment of the method, the three-dimensional face geometry at least approximates a geometry of a human face (or at least of the mouth region), wherein an image plane comprising at least the boundaries of the inner mouth region is projected onto the three-dimensional geometry. In particular, the three-dimensional face geometry may comprise at least a part of a side surface of a cylinder or prism, onto which part of the side surface an image plane of at least the part of the image comprising the mouth region is projected.

According to one embodiment of the method, in the step of computing the lighting the multitude of surface points of the dental model are vertices of the dental model.

According to another embodiment of the method, visualizing the dental model in the two-dimensional image also comprises adapting a colouring of the dental model based on a colouring of the image. Adapting the colouring comprises identifying, for at least one of teeth, gum and lips, a reference colour in the image. Said reference colour is an average colour, and identifying a reference colour comprises performing an automatic segmentation of image pixels relating to teeth, gum and/or lips in the image. The reference colour or reference colours are used for estimating a colour space of the image. The estimated colour space of the image is used to transform a colour space of the dental model. In particular, the estimated colour space of the image is used as colour space of the dental model.

According to another embodiment of the method, the lighting of the dental model is computed based on an estimated lighting situation. The lighting situation can be estimated based on the lighting of the face in the image. Estimating the lighting situation may in particular comprise
- estimating low frequency lighting, particularly with spherical harmonics or virtual point lights, and/or
- detecting reflections in eyes of the face to estimate a position of light sources, particularly high frequency lighting.

According to another embodiment of the method, a soft or blurred edge of the inner mouth region is calculated from the determined boundary, wherein computing the shadowing is based on the soft or blurred edge in order to avoid hard shadow edges.

According to another embodiment of the method, computing the lighting of the dental model comprises applying lighting and shading effects, the effects comprising at least one of specular reflections, colours and ambient occlusion.

According to another embodiment of the method, visualizing the dental model comprises simulating camera effects to approximate an appearance of the dental model to the appearance of the face in the image, the simulated camera effects comprising at least one of dynamic range, contrast, saturation, noise, lens distortion, white balance, defocus blur and motion blur.

According to another embodiment of the method, the image is part of an image stream, and the method is performed in real time for at least a multitude of images of the image stream.

According to one embodiment, visualizing the dental model in the two-dimensional image comprises adapting a colouring of the dental model based on a colouring of a plurality of images of the image stream, particularly comprising the actual image and previous images of the image stream, in particular wherein mean values from a plurality of consecutive images are used for adapting the colouring.

According to another embodiment, the method further comprises capturing the image stream by means of a camera and visualizing the dental model and the two-dimensional image on a displaying device to a user, particularly wherein the face is the face of the user.

According to a further embodiment, the camera and the displaying device are part of the same mobile device, wherein the method is performed by means of one or more algorithms installed on a computing unit of the mobile device.

According to another embodiment of the method, the virtual dental model is in a polygon mesh format and comprises a plurality of vertices. Computing the lighting of the dental model optionally comprises computing a shading. This computing of the shading may be performed in a vertex shader and/or locally per vertex or per fragment.

A second aspect of the invention pertains to a mobile device comprising a camera and a display that arranged so that images of a user's face are capturable by the camera while the user watches the display - i.e. for instance a Smartphone having a "selfie camera". The device comprises a computing unit with at least one algorithm that is adapted to perform the method according to the first aspect. In particular, the image with the overlaid visualization of the model is displayable on the display.

A further aspect of the invention pertains to a computer programme product comprising programme code which is stored on a machine-readable medium, or being embodied by an electromagnetic wave comprising a programme code segment, and having computer-executable instructions for performing the method according to the first aspect.

The invention in the following will be described in detail by referring to exemplary embodiments that are accompanied by figures, in which:
- Fig. 1: illustrates an exemplary embodiment of a computer-implemented method for realistic visualization of a 3D dental model in an image of a person's face;
- Fig. 2: shows an inner mouth area identified in the image;
- Fig. 3: illustrates determining lighting of the dental model not covered by the present claims;
- Fig. 4: illustrates determining lighting of the dental model not covered by the present claims; and
- Fig. 5: illustrates determining realistic lighting of the dental model according to the presently claimed invention.

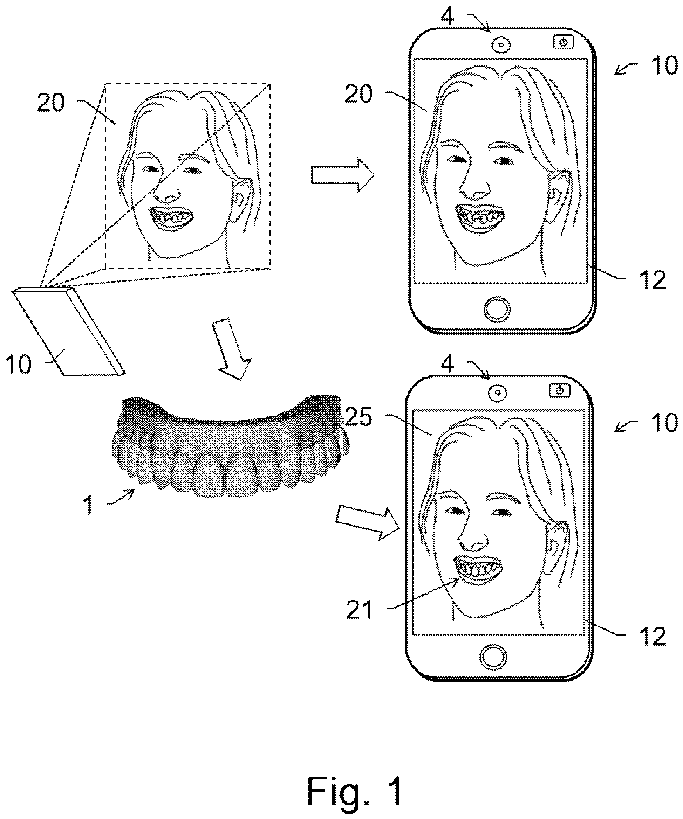

Figure 1 illustrates a computer-implemented method for realistic visualization of a 3D dental model in an image of a person's face according to an exemplary embodiment of the invention.

According to the shown embodiment, an image 20 of the person's face is captured by means of a camera 4. In this embodiment, the camera 4 is part of a handheld mobile device 10 such as a smartphone. The device 10 comprises a display 12 which is enabled to display the image 20 in real time to the user, i.e. the person whose image is captured. Data of a three-dimensional (3D) virtual dental model 1 is provided in a data storage of the mobile device 10. An algorithm being provided in a computing unit of the mobile device 10 is adapted to fit the dental model 1 into the image to provide an augmented-reality (AR) image 25 on the display 12, wherein the rendered dental model 21 (e. g. a 2D visualization of the model) is displayed in the inner mouth region of the face. The processing can be performed offline on an image or video or in real time on a live camera image thus providing a live augmented-reality application.

In some embodiments, the dental model 1 may comprise appliances that are attached - either temporarily or permanently - to dentures or real teeth of the dental model 1, such as dental braces and retainers or mouthguards for several purposes. In some embodiments the dental model 1 comprises only these appliances or only the appliances of the model are visualized in the image.

In the field of artificial dentition, the rendering of digital dental models 1 without realistic lighting information leads to unsatisfactory results. Realistic visualization is important in this field, as humans are very sensitive to even slight deviations from reality in human faces. To overcome this "uncanny valley" problem in a visualization of virtual teeth in a photo or video of a face, realistic lighting of the 3D rendered content is crucial. According to the disclosed invention, the realism may be achieved by rendering of shadows and optionally by also applying colour correction, so that the lighting of the rendered virtual content should match the lighting of the real scene - i.e. essentially the face.

For calculating a shadowing, in one embodiment, a given or constant lighting situation simply may be assumed. For more realistic results, alternatively, an approximate lighting situation in the image can be detected. The lighting can be estimated using the face as a light probe to obtain an approximation of the lighting of the face. Both, low frequency lighting - e. g. with spherical harmonics or virtual point lights - and high frequency lighting for reflections (e. g. from the eyes) can be estimated.

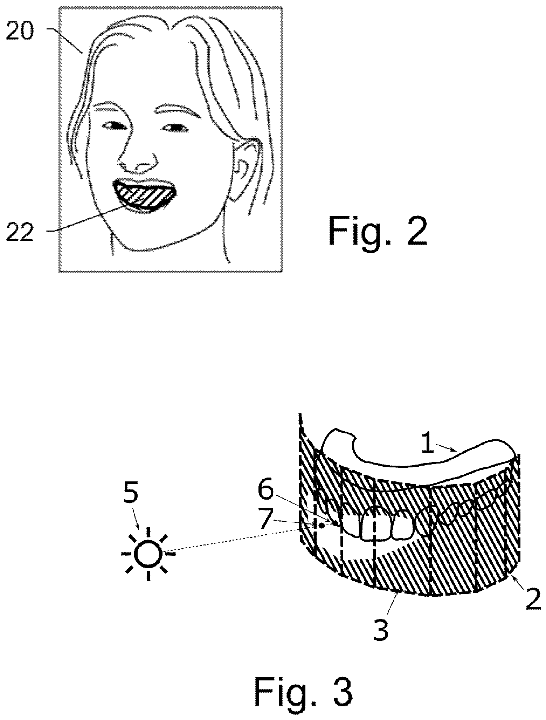

According to the present invention, shadowing can be calculated even without having a detailed 3D model of the face. According to some embodiments of the invention, the 2D image of an inner mouth region 22 (e. g. a binary 2D mouth mask image) is used as a 3D occluder. In these embodiments, as illustrated in Figure 2, adding realistic lighting to the dental model 1 comprises identifying the boundaries of the inner mouth region 22 in the imaged face. In the exemplary embodiment of Figure 2, the inner mouth region 22 comprises that part of the imaged face that lies inside the lips. The inner mouth region 22 can either be defined by the user, e. g. by selecting the area in the image 20, or be automatically identified by means of a feature recognition algorithm.

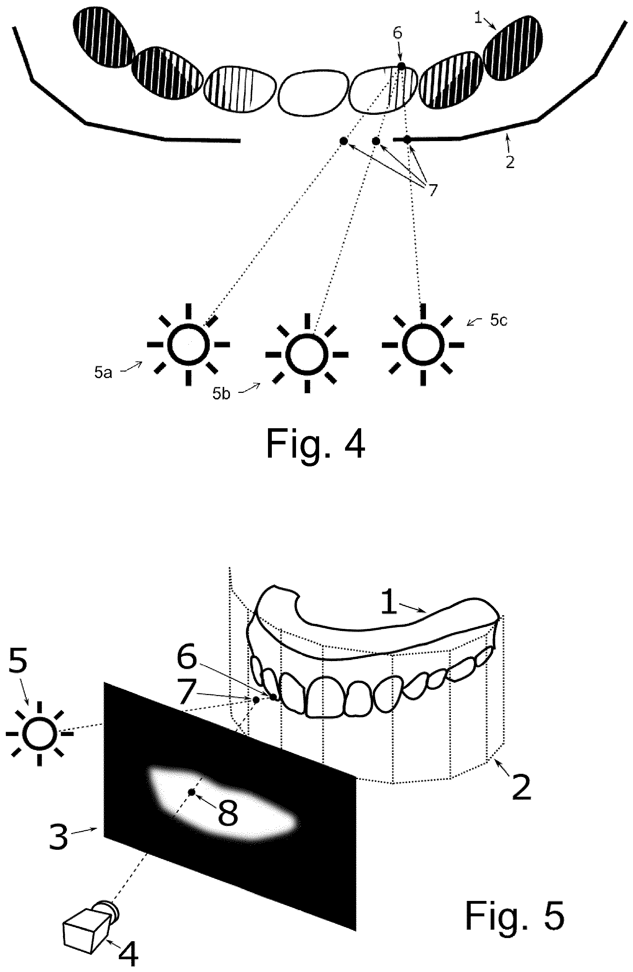

Having identified the boundaries of the inner mouth region 22, these can be used to calculate light and shadow in the area behind the inner mouth region 22, i.e. the area where the dentition corresponding to the virtual model 1 would be placed in reality. The method makes use of a 3D mouth contour to cast shadows. Optionally, this is followed by applying a colour correction to match the camera image properties such as white balance, brightness, saturation, contrast etc. This is illustrated in the following Figures 3, 4 and 5, wherein the methods illustrated in Figs. 3 and 4 are not part of the presently claimed subject matter.

If the 2D image is captured together with depth information, e. g. using a depth sensor, the available 3D information of the face can be used to provide the needed 3D mouth contour.

If only a two-dimensional (2D) mouth region is available from the 2D image, the mouth region and the surrounding part of the face need to be brought to 3D. To do so, according to the embodiment of the method illustrated in Figure 3, at least the inner mouth region or a shape thereof is projected onto a 3D geometry 2 that approximates a geometry of a human face. In particular, the geometry 2 may be the side face of a cylinder or prism. Alternatively, a 3D facial model with explicit face geometry can be generated to cast shadows. The known 2D boundaries of the inner mouth region 22 that has been projected to 3D can then be used for calculating 3D lighting and shadowing.

Having assumed or estimated a position of a light source 5 relative to the face in the image, its position relative to each point of the projection 3 of the inner mouth region can be determined. The model 1 is positioned behind the mouth region in a position the real dentition would be placed in a real mouth. Assuming that the 3D pose of the virtual dental model 1 is known, e. g. based on deep learning approaches or facial landmarks with a 3D head pose estimation, the camera parameters to render the 3D model 1 are given. Accordingly, the model 1 can be positioned behind the projection with the correct pose.

For a multitude of points 6 of the model 1, it is then determined whether an interjection 7 of a ray originating in the light source 5 with the 3D geometry 2 lies within the inner mouth region. If it does, the point 6 is lighted by the light source 5, if not, the point is shadowed by the face. If the virtual dental model 1 is in a polygon mesh format the points can be vertices or fragments. The lighting of the virtual dental model 1 can be computed locally to take both the estimated lighting as well as the shadowing from the exterior of the mouth region into account.

The boundaries of the defined mouth region optionally can be embedded as soft or blurred edges to avoid unrealistic hard shadow edges, especially if point-shaped virtual lights sources are used. In addition, other lighting and shading can be applied, such as specular reflections, colours or ambient occlusion. Optionally, further camera effects can be simulated to provide a more realistic appearance of the rendered model. The camera effects comprise, for instance, dynamic range, contrast, saturation, noise, lens distortion, white balance, defocus or motion blur.

Figure 4 illustrates another method not covered by the present claims, showing the dental model 1 and 3D geometry 2 in a cross sectional view. For a point 6 on the surface of the model 1, intersections 7 of three rays originating from three light sources 5a-c are tested on their position inside or outside the inner mouth region. Here, the intersections 7 of rays from light sources 5a and 5b are inside the inner mouth region, and the intersection 7 related to light source 5c is not. This means that point 6 is lighted only by two of the three light sources (i.e. light sources 5a and 5b).

The model 1 has four different brightness levels. These depend on how many of the three light sources 5a-c illuminate a point or portion of the model 1. The highest brightness is in the centre of the model, where all three light sources 5a-c illuminate the surface of the dental model 1. Point 6 lies in an area having the second brightness level. The third brightness level is illuminated only by one of the light sources 5a-c, and the fourth brightness level is completely shadowed by the face when projected on the 3D geometry.

It is important to provide the correct brightness to each of the several brightness levels, i.e. not to visualize the rendered model or parts thereof too dark or too bright to look realistic. Therefore, the method may optionally comprise a step of detecting a lighting situation of the mouth background in the image, e. g. by how far the inside of the mouth cavity is darker than the outside of the face. To do so, the darkest point of the mouth background may be determined to serve as a reference for the shadowing. Additionally or alternatively, a contrast level of the camera image may be detected.

Figure 5 illustrates an embodiment of a method of determining lighting for the dental model 1 according to the present invention. This embodiment comprises performing the lighting computation in the vertex shader. For each vertex 6 a ray to each virtual point light source 5 is generated. Its intersection 7 with the face proxy geometry 2 (e. g. a cylinder) is used to create a ray to the (virtual) camera 4. Its projection 8 into the image plane 3 is the lookup location to determine whether the light is visible through the inner mouth region, i.e. not occluded by lips or facial tissue.

Having added lighting information to the model, the rendered 3D model is overlaid on the photo only within the defined mouth region. Also, colours can be adapted such that the 3D rendered content matches the camera image. One way to match colours is to automatically find an average teeth colour and/or an average gum or lip colour in the image. They can be obtained from an automatic segmentation of tooth pixels and gum and lip pixels. These average colours serve as reference colours and can be used to transform the colour space to the one estimated from the camera image. Of course, instead of using average colours, reference colours can also be derived using minimum and/or maximum values or histograms.

If a video is rendered instead of a single image, to avoid flickering over time, reference colours from previous frames can be taken into account, e. g. by temporally smoothing the values.

According to the present invention a method is provided for visualization of a virtual dental model in an image of a face. The method comprises obtaining a virtual dental model. The virtual dental model includes a three-dimensional representation of a dental object to be visualized, for example, a denture or other dental prosthesis, braces, mouth guard, jewellery, etc. An assumed, measured, or approximated lighting situation of the image is determined. For example, the method may include determining a position of at least one light source of the image used to illuminate the face of the image. An inner mouth region in the image is determined wherein the inner mouth region is defined by inner boundaries of lips of the face. In some embodiments, the inner mouth region is determined using a processor. A processor is used to compute a shadow in the inner mouth region based on a face geometry and the determined lighting situation (e.g., the position of the at least one light source). The face geometry may be, for example, an approximated face geometry. A lighting of the dental model is computed using the processor, wherein computing of the lighting takes into account shadowing in the inner mouth region in the manner as defined in claim 1. The dental model is displayed on a display with the computed lighting applied and wherein the dental model is shown within the inner mouth region of the image.

In the context of the present invention, the term "denture" is not necessarily restricted to full dentures but also comprises partial dentures or orthodontic situation/adaptations or dental restorations such as dental prostheses, including crowns, crown lays, veneers, inlays and onlays, bridges, dental implants, implant restorations. Accordingly, the term "dental model" includes all models of dental prostheses as well as the patient situation that could be partial or fully edentulous - such as models of complete and partial dentures - that are used for prosthodontic purposes.

A method according to the present invention may be carried out using a system having a camera, a processor, an electronic data storage unit, and a display. The camera can be a standard camera, an infrared dot-projection detector, flood illuminator camera, structured-light three-dimensional scanner, standard infrared detector, ultrasonic imaging device, Doppler detector, or any other suitable visualization system capable of capturing information related to a patient's dentition. The processor can be a single processor having one or more cores, or a plurality of processors connected by a bus, network, or other data link. The electronic data storage unit can be any form of non-transitory computer-readable storage medium suitable for storing the data produced by the system. The display can be any display suitable for displaying a digital color or grayscale image.

The camera, processor, electronic data storage unit, and digital display are components of a single device. The single device may be a smartphone, tablet, laptop computer, personal digital assistant, or other computing device.

A method according to the present invention may be carried out with the processor being in communication over a network, which could be wired or wireless, with an external processor used for performing one or more calculation steps and/or a network-attached electronic data storage unit. In some embodiments, the method of the present invention makes use of cloud computing to perform one or more calculations steps remotely and/or remote storage to enable the storage of data remotely for collaborative or remote analysis. A method according to the present invention may make use of a system which comprises a plurality of graphical user interfaces to permit multiple users to view or analyze the same data.

A method according to the present invention may utilize a system which operates to provide one or more users with a visualization of a virtual dental model of a patient's teeth, which may be altered to visualize the effect of one or more dental or orthodontic alterations. This allows the one or more users to visualize a "before" dentition image, i.e., the appearance of a patient's dentition prior to a dental or orthodontic procedure, and an "after" dentition image, i.e., a representation of the expected appearance of a patient's dentition after a proposed dental or orthodontic procedure.

A method according to the present invention may utilize a system which operates by capturing information related to a patient's dentition using a camera, creating a model of the patient's dentition on a processor, fitting a model of a proposed post-alteration dentition to the patient's dentition on the processor, coloring the model of the proposed post-alteration dentition to match an expected real post-alteration coloration, and displaying the fitted model of the proposed post-alteration dentition in place of the patient's actual dentition on a display which otherwise shows the patient's actual facial features. The information related to a patient's dentition, the model of the patient's dentition, and the model of the proposed post-alteration dentition may be stored on an electronic data storage unit. In some embodiments, the operations are performed in real-time.

A method according to the present invention may be implemented with a user interface which is configured such that a user may view the "before" dentition image and the "after" dentition image simultaneously either side-by-side or with a full or partial overlay.

Where used herein, the term "non-transitory" is a limitation on the computer-readable storage medium itself-that is, it is tangible and not a signal-as opposed to a limitation on the persistence of data storage. A non-transitory computer-readable storage medium does not necessarily store information permanently. Random access memory (which may be volatile, non-volatile, dynamic, static, etc.), read-only memory, flash memory, memory caches, or any other tangible, computer-readable storage medium, whether synchronous or asynchronous, embodies it.

## Claims

1. Method for visualization of a virtual dental model (1) in a two-dimensional image (20) of a face, the image comprising at least an inner mouth region (22) of the face, the dental model (1) comprising a three-dimensional representation of a denture or other dental prosthesis, wherein the method comprises: determining boundaries of the inner mouth region (22) in the image, particularly wherein the boundaries are defined by inner boundaries of lips of the face, **characterized in that** the method further comprises:
- estimating or assuming a lighting situation in the image (20) of the face, the lighting situation comprising one or more virtual point light sources (5),
- computing, based on the boundaries of the inner mouth region (22), on a three-dimensional face geometry (2) and on the lighting situation, a lighting of the dental model (1) taking into account shadowing in the inner mouth region (22) by
generating, for a multitude of surface points (6) on the surface of the virtual dental model (1), a first ray to each of the one or more virtual point light sources (5),
generating for each first ray a second ray to a virtual camera (4), using an intersection (7) of the first ray with the three-dimensional face geometry (2) as a starting point for the second ray, and
using an intersection (8) of the second ray with an image plane (3) as a lookup location to determine a lighting for the respective surface point (6), wherein a surface point (6) is assumed to be lighted by a virtual point light source (5) if the second ray intersects the image plane (3) at the inner mouth region (22), and
- visualizing the dental model (1) and the two-dimensional image (20), wherein a visualization (21) of the dental model (1) has the computed lighting.

2. Method according to claim 1,
**characterized in that**
the three-dimensional face geometry (2) comprises a geometry of the face and is acquired using a depth sensor, particularly wherein the three-dimensional face geometry (2) is acquired together with the two-dimensional image (20) of the face and/or as a range image.

3. Method according to claim 1,
**characterized in that**
the three-dimensional face geometry (2) at least approximates a geometry of a human face, wherein an image plane (3) comprising at least the boundaries of the inner mouth region (22) is projected onto the three-dimensional geometry (2), particularly wherein the three-dimensional face geometry (2) comprises at least a part of a side surface of a cylinder or prism, onto which part of the side surface an image plane (3) of at least the part of the image comprising the mouth region is projected.

4. Method according to claim 1,
**characterized in that**
in the step of computing the lighting of the dental model the multitude of surface points (6) are vertices of the virtual dental model (1).

5. Method according to any one of the preceding claims,
**characterized in that**
visualizing the dental model (1) in the two-dimensional image (20) comprises adapting a colouring of the dental model (1) based on a colouring of the image (20), particularly wherein adapting the colouring comprises
- identifying, for at least one of teeth, gum and lips, a reference colour in the image (20), particularly wherein
- the reference colour is an average colour, and/or
- identifying a reference colour comprises performing an automatic segmentation of image pixels relating to teeth, gum and/or lips in the image (20),
- using the reference colour or reference colours for estimating a colour space of the image (20), and
- using the colour space of the image (20) to transform a colour space of the dental model (1), particularly wherein the colour space of the image (20) is used for the colour space of the dental model (1).

6. Method according to any one of the preceding claims,
**characterized in that**
the lighting of the dental model (1) is computed based on an estimated lighting situation, wherein the lighting situation is estimated based on the lighting of the face in the image (20), particularly wherein estimating the lighting situation comprises
- estimating low frequency lighting, particularly with spherical harmonics or virtual point lights, and/or
- detecting reflections in eyes of the face to estimate a position of light sources, particularly high frequency lighting.

7. Method according to any one of the preceding claims,
**characterized in that**
a soft or blurred edge of the inner mouth region (22) is calculated from the determined boundary, wherein computing the shadowing is based on the soft or blurred edge in order to avoid hard shadow edges.

8. Method according to any one of the preceding claims,
**characterized in that**
- computing the lighting of the dental model (1) comprises applying lighting and shading effects, the effects comprising at least one of specular reflections, colours and ambient occlusion; and/or
- visualizing the dental model comprises simulating camera effects to approximate an appearance of the dental model (1) to the appearance of the face in the image (20), the simulated camera effects comprising at least one of dynamic range, contrast, saturation, noise, lens distortion, white balance, defocus blur and motion blur.

9. Method according to any one of the preceding claims,
**characterized in that**
the image (20) is part of an image stream, and the method is performed in real time for at least a multitude of images (20) of the image stream.

10. Method according to claim 9,
**characterized in that**
visualizing the dental model (1) in the two-dimensional image (20) comprises adapting a colouring of the dental model (1) based on a colouring of a plurality of images (20) of the image stream, particularly comprising the actual image and previous images of the image stream, in particular wherein mean values from a plurality of consecutive images are used for adapting the colouring.

11. Method according to claim 9 or claim 10,
**characterized in that**
the method further comprises capturing the image stream by means of a camera (4) and visualizing the dental model (1) and the two-dimensional image (20) on a displaying device (12) to a user, particularly wherein the face is the face of the user.

12. Method according to any one of claims 9 to 11,
**characterized in that**
the camera (4) and the displaying device (12) are part of the same mobile device (10), wherein the method is performed by means of one or more algorithms installed on a computing unit of the mobile device (10).

13. Method according to any one of the preceding claims,
**characterized in that**
the virtual dental model (1) is in a polygon mesh format and comprises a plurality of vertices, particularly wherein computing the lighting of the dental model comprises computing a shading, wherein computing the shading is performed in a vertex shader and/or locally per vertex or per fragment.

14. Mobile device (10) comprising a camera (4) and a display (12) that arranged so that images (20) of a user's face are capturable by the camera (4) while the user watches the display (12),
**characterized in that**
the device comprises a computing unit with at least one algorithm that is adapted to perform the method of one of the preceding claims.

15. Computer programme product comprising programme code which is stored on a machine-readable medium and having computer-executable instructions for performing the method according to any one of claims 1 to 13.

## Patentansprüche

1. Verfahren zur Visualisierung eines virtuellen Dentalmodells (1) in einem zweidimensionalen Bild (20) eines Gesichts, wobei das Bild wenigstens eine innere Mundregion (22) des Gesichts enthält, wobei das Dentalmodell (1) eine dreidimensionale Darstellung eines künstlichen Gebisses oder einer anderen Dentalprothese umfasst, wobei das Verfahren beinhaltet: Grenzen der inneren Mundregion (22) in dem Bild zu bestimmen, wobei die Grenzen insbesondere durch innere Grenzen der Lippen des Gesichts definiert sind, **dadurch gekennzeichnet, dass** das Verfahren weiter beinhaltet,
- im Bild (20) des Gesichts eine Beleuchtungssituation abzuschätzen oder anzunehmen, wobei die Beleuchtungssituation eine oder mehrere virtuelle Punktlichtquellen (5) beinhaltet,
- basierend auf den Grenzen der inneren Mundregion (22), auf einer dreidimensionalen Gesichtsgeometrie (2) und auf der Beleuchtungssituation eine Beleuchtung des Dentalmodells (1) zu berechnen, die Schattenbildung in der inneren Mundregion (22) berücksichtigt, indem
für eine Mehrzahl von Oberflächenpunkten (6) an der Oberfläche des virtuellen Dentalmoduls (1) ein erster Strahl zu jeder von der einen oder den mehreren Punktlichtquellen (5) erzeugt wird,
für jeden ersten Strahl ein zweiter Strahl zu einer virtuellen Kamera (4) unter Verwendung eines Schnittpunkts (7) des ersten Strahls mit der dreidimensionalen Gesichtsgeometrie (2) als Startpunkt für den Strahl erzeugt wird und
ein Schnittpunkt (8) des zweiten Strahls mit einer Bildebene (3) als Zuordnungsortspunkt verwendet wird, um eine Beleuchtung des jeweiligen Oberflächenpunkts (6) zu bestimmen, wobei ein Oberflächenpunkt (6) als durch eine virtuelle Punktlichtquelle (5) beleuchtet angenommen wird, wenn der zweite Strahl die Bildebene (3) in der inneren Mundregion (22) schneidet, und
- das Dentalmodell (1) und das zweidimensionale Bild (20) zu visualisieren, wobei eine Visualisierung (21) des Dentalmodells (1) die berechnete Beleuchtung hat.

2. Verfahren nach Anspruch 1,
**dadurch gekennzeichnet, dass**
die dreidimensionale Gesichtsgeometrie (2) eine Geometrie des Gesichts umfasst und unter Verwendung eines Tiefensensors aufgenommen ist, wobei insbesondere die dreidimensionale Gesichtsgeometrie (2) zusammen mit dem zweidimensionalen Bild (20) des Gesichts und/oder als ein Entfernungsbild aufgenommen wird.

3. Verfahren nach Anspruch 1,
**dadurch gekennzeichnet, dass**
die dreidimensionale Gesichtsgeometrie (2) eine Geometrie eines menschlichen Gesichtes wenigstens approximiert, wobei eine Bildebene (3), die wenigstens die Grenzen der inneren Mundregion (22) enthält, auf die dreidimensionale Geometrie (2) projiziert wird, wobei die dreidimensionale Gesichtsgeometrie (2) insbesondere wenigstens einen Teil einer Seitenfläche eines Zylinders oder Prismas beinhaltet, wobei auf den Teil der Seitenfläche eine Bildebene (3) wenigstens des Teils des Bildes, der die Mundregion enthält, projiziert wird.

4. Verfahren nach Anspruch 1,
**dadurch gekennzeichnet, dass**
im Schritt der Berechnung der Beleuchtung des Dentalmodells die Mehrzahl von Oberflächenpunkten (6) Vertices des virtuellen Dentalmodells (1) sind.

5. Verfahren nach einem der vorhergehenden Ansprüche,
**dadurch gekennzeichnet, dass**
die Visualisierung des Dentalmodells (1) im zweidimensionalen Bild (20) beinhaltet, eine Färbung des Dentalmodells (1) basierend auf einer Färbung des Gesichts (20) anzupassen, wobei insbesondere das Anpassen der Färbung beinhaltet,
- für wenigstens eines von Zähnen, Zahnfleisch und Lippen eine Referenzfarbe im Bild (20) identifiziert wird, wobei insbesondere
- die Referenzfarbe eine mittlere Farbe ist und/oder
- das Identifizieren einer Bezugfarbe beinhaltet, eine automatische Segmentierung von Bildpixeln, die sich auf Zähne, Zahnfleisch und/oder Lippen im Bild (20) beziehen, durchzuführen,
- die Bezugsfarbe oder die Referenzfarben verwendet werden, um einen Farbraum des Bildes (20) abzuschätzen und
- den Farbraum des Bildes (20) zu verwenden, um einen Farbraum des Dental modells (1) zu transformieren, wobei insbesondere der Farbraum des Bildes (20) für den Farbraum des Dentalmodells (1) verwendet wird.

6. Verfahren nach einem der vorhergehenden Ansprüche,
**dadurch gekennzeichnet, dass**
die Beleuchtung des Dentalmodells (1) basierend auf einer geschätzten Beleuchtungssituation berechnet wird, wobei die Beleuchtungssituation basierend auf der Beleuchtung des Gesichts in dem Bild (20) geschätzt wird, wobei das Schätzen der Beleuchtungssituation insbesondere beinhaltet
- eine niederfrequente Beleuchtung zu schätzen, insbesondere mit Kugelflächenfunktionen oder virtuellen Punktlichtquellen und/oder
- Reflexionen in den Augen des Gesichts zu detektieren, um eine Position von Lichtquellen zu schätzen, insbesondere hochfrequente Beleuchtung.

7. Verfahren nach einem der vorhergehenden Ansprüche,
**dadurch gekennzeichnet, dass**
aus der bestimmten Grenze ein weicher oder unscharfer Rand der inneren Mundregion (22) berechnet wird, wobei die Berechnung der Abschattung auf dem weichen oder unscharfen Rand basiert, um harte Schattenränder zu vermeiden.

8. Verfahren nach einem der vorhergehenden Ansprüche,
**dadurch gekennzeichnet, dass**
- das Berechnen der Beleuchtung des Dentalmodells (1) beinhaltet, Beleuchtungs- und Schatteneffekte anzuwenden, wobei die Effekte wenigstens eines umfassen von Reflexionen, Farben und Umgebungsverdeckung, und/oder
- das Visualisieren des Dentalmodells die Simulierung von Kameraeffekten beinhaltet, um ein Erscheinungsbild des Dentalmodells an das Erscheinungsbild des Gesichts im Bild (20) zu approximieren, wobei die simulierten Kameraeffekte wenigstens eines umfassen von: dynamischer Bereich, Kontrast, Sättigung, Rauschen, Linsenverzerrung, Weißabgleich, Defokussierungsunschärfe und Bewegungsunschärfe.

9. Verfahren nach einem der vorhergehenden Ansprüche,
**dadurch gekennzeichnet, dass**
das Bild (20) Teil eines Bildstroms ist und dass das Verfahren in Echtzeit für wenigstens eine Mehrzahl von Bildern (20) des Bildstroms durchgeführt wird.

10. Verfahren nach Anspruch 9,
**dadurch gekennzeichnet, dass**
das Visualisieren des Dentalmodells (1) im zweidimensionalen Bild (20) beinhaltet, eine Färbung des Dentalmodells (1) basierend auf einer Färbung der Mehrzahl von Bildern (20) des Bildstroms anzupassen, insbesondere auf dem aktuellen Bild und den vorhergehenden Bildern des Bildstroms, wobei insbesondere Mittelwerte von einer Mehrzahl von aufeinanderfolgenden Bildern verwendet werden, um die Färbung anzupassen.

11. Verfahren nach Anspruch 9 oder Anspruch 10,
**dadurch gekennzeichnet, dass**
das Verfahren weiter beinhaltet, den Bildstrom mittels einer Kamera (4) aufzunehmen und das Dentalmodell (1) und das zweidimensionale Bild (20) auf einer Anzeigevorrichtung (12) für einen Benutzer zu visualisieren, wobei insbesondere das Gesicht das Gesicht des Benutzer ist.

12. Verfahren nach einem der Ansprüche 9-11,
**dadurch gekennzeichnet, dass**
die Kamera (4) und die Anzeigevorrichtung (12) Teil derselben mobilen Vorrichtung (10) sind, wobei das Verfahren mittels eines oder mehrerer Algorithmen durchgeführt wird, die in einer Computereinheit der mobilen Vorrichtung (10) installiert sind.

13. Verfahren nach einem der vorhergehenden Ansprüche,
**dadurch gekennzeichnet, dass**
das virtuelle Dentalmodell (1) in einem Polygonnetzformat vorliegt und eine Mehrzahl von Vertices enthält, wobei insbesondere die Berechnung der Beleuchtung des Dentalmodells beinhaltet, eine Schattierung zu berechnen, wobei die Berechnung der Schattierung in einem Vertex-Shader und/oder lokal pro Vertex oder pro Fragment durchgeführt wird.

14. Mobile Vorrichtung (10) mit einer Kamera (4) und einer Anzeige (12), die dazu ausgestaltet sind, so dass Bilder (20) des Gesichts eines Benutzers durch die Kamera (4) aufnehmbar sind, während der Benutzer die Anzeige (12) betrachtet,
**dadurch gekennzeichnet, dass**
die Vorrichtung eine Computereinheit mit wenigstens einem Algorithmus enthält, der dazu eingerichtet ist, um das Verfahren nach einem der vorhergehenden Ansprüche auszuführen.

15. Computer-Programmprodukt, das einen Programmcode enthält, der auf einem maschinenlesbaren Medium gespeichert ist und der computerausführbare Instruktionen zur Ausführung des Verfahrens nach einem der Ansprüche 1-13 enthält.

## Revendications

1. Procédé de visualisation d'un modèle dentaire virtuel (1) dans une image bidimensionnelle (20) d'un visage, l'image comprenant au moins une région de bouche interne (22) du visage, le modèle dentaire (1) comprenant une représentation tridimensionnelle d'un dentier ou d'une autre prothèse dentaire, dans lequel le procédé comprend l'étape consistant à : déterminer des limites de la région de bouche interne (22) dans l'image, en particulier dans lequel les limites sont définies par des limites internes de lèvres du visage, **caractérisé en ce que** le procédé comprend en outre les étapes consistant à:
- estimer ou supposer une situation d'éclairage dans l'image (20) du visage, la situation d'éclairage comprenant une ou plusieurs sources lumineuses ponctuelles virtuelles (5),
- calculer, sur la base des limites de la région de la bouche interne (22), d'une géométrie de visage en trois dimensions (2) et de la situation d'éclairage, un éclairage du modèle dentaire (1) prenant en compte l'ombrage dans la région de bouche interne (22) en
générant, pour une multitude de points de surface (6) sur la surface du modèle dentaire virtuel (1), un premier rayon vers chacune des une ou plusieurs sources lumineuses ponctuelles virtuelles (5),
générant pour chaque premier rayon un second rayon vers une caméra virtuelle (4), en utilisant une intersection (7) du premier rayon avec la géométrie de visage tridimensionnelle (2) comme point de départ pour le second rayon, et
utilisant une intersection (8) du second rayon avec un plan d'image (3) comme emplacement de consultation pour déterminer un éclairage pour le point de surface respectif (6), dans lequel un point de surface (6) est supposé être éclairé par une source lumineuse ponctuelle virtuelle (5) si le second rayon recoupe le plan d'image (3) au niveau de la région de bouche intérieure (22), et
- visualiser le modèle dentaire (1) et l'image bidimensionnelle (20), dans lequel une visualisation (21) du modèle dentaire (1) présente l'éclairage calculé.

2. Procédé selon la revendication 1,
**caractérisé en ce que**
la géométrie de visage tridimensionnelle (2) comprend une géométrie du visage et est acquise en utilisant un capteur de profondeur, en particulier dans lequel la géométrie de visage tridimensionnelle (2) est acquise conjointement avec l'image bidimensionnelle (20) du visage et/ou en tant qu'image de distance.

3. Procédé selon la revendication 1,
**caractérisé en ce que**
la géométrie de visage tridimensionnelle (2) se rapproche au moins d'une géométrie d'un visage humain, dans lequel un plan d'image (3) comprenant au moins les limites de la région de bouche intérieure (22) est projeté sur la géométrie tridimensionnelle (2), en particulier dans lequel la géométrie de visage tridimensionnelle (2) comprend au moins une partie d'une surface latérale d'un cylindre ou d'un prisme, partie de la surface latérale sur laquelle un plan d'image (3) d'au moins la partie de l'image comprenant la région de bouche est projeté.

4. Procédé selon la revendication 1,
**caractérisé en ce que**
dans l'étape de calcul de l'éclairage du modèle dentaire, la multitude de points de surface (6) sont des sommets du modèle dentaire virtuel (1).

5. Procédé selon l'une quelconque des revendications précédentes,
**caractérisé en ce que**
la visualisation du modèle dentaire (1) dans l'image bidimensionnelle (20) comprend une adaptation d'une coloration du modèle dentaire (1) sur la base d'une coloration de l'image (20), en particulier dans lequel l'adaptation de la coloration comprend les étapes consistant à
- identifier, pour au moins l'une des dents, la gencive et les lèvres, une couleur de référence dans l'image (20), en particulier dans lequel
- la couleur de référence est une couleur moyenne, et/ou
- l'identification d'une couleur de référence comprenant la réalisation d'une segmentation automatique de pixels d'image relatifs aux dents, aux gencives et/ou aux lèvres dans l'image (20),
- utiliser la couleur de référence ou les couleurs de référence pour estimer un espace colorimétrique de l'image (20), et
- utiliser l'espace colorimétrique de l'image (20) pour transformer un espace colorimétrique du modèle dentaire (1), en particulier dans lequel l'espace colorimétrique de l'image (20) est utilisé pour l'espace colorimétrique du modèle dentaire (1).

6. Procédé selon l'une quelconque des revendications précédentes,
**caractérisé en ce que**
l'éclairage du modèle dentaire (1) est calculé sur la base d'une situation d'éclairage estimée, dans laquelle la situation d'éclairage est estimée sur la base de l'éclairage du visage dans l'image (20), en particulier dans lequel l'estimation de la situation d'éclairage comprend les étapes consistant à
- estimer un éclairage basse fréquence, en particulier avec des harmoniques sphériques ou des lumières ponctuelles virtuelles, et/ou
- détecter des réflexions dans les yeux du visage pour estimer une position des sources lumineuses, en particulier un éclairage haute fréquence.

7. Procédé selon l'une quelconque des revendications précédentes,
**caractérisé en ce que**
un bord doux ou flou de la région de bouche interne (22) est calculé à partir de la limite déterminée, dans lequel le calcul de l'ombrage est basé sur le bord doux ou flou afin d'éviter des bords d'ombre durs.

8. Procédé selon l'une quelconque des revendications précédentes,
**caractérisé en ce que**
- le calcul de l'éclairage du modèle dentaire (1) comprend une application d'effets d'éclairage et d'ombrage, les effets comprenant au moins une parmi des réflexions spéculaires, des couleurs et une occlusion ambiante; et/ou
- la visualisation du modèle dentaire comprend une simulation d'effets de caméra pour rapprocher une apparence du modèle dentaire (1) de l'apparence du visage dans l'image (20), les effets de caméra simulés comprenant au moins un parmi une plage dynamique, un contraste, une saturation, un bruit, une distorsion de lentille, une balance des blancs, un flou de défocalisation et un flou de mouvement.

9. Procédé selon l'une quelconque des revendications précédentes,
**caractérisé en ce que**
l'image (20) fait partie d'un flux d'images, et le procédé est mis en œuvre en temps réel pour au moins une multitude d'images (20) du flux d'images.

10. Procédé selon la revendication 9,
**caractérisé en ce que**
la visualisation du modèle dentaire (1) dans l'image bidimensionnelle (20) comprend une adaptation d'une coloration du modèle dentaire (1) sur la base d'une coloration d'une pluralité d'images (20) du flux d'images, comprenant en particulier l'image réelle et des images précédentes du flux d'images, en particulier dans lequel des valeurs moyennes d'une pluralité d'images consécutives sont utilisées pour adapter la coloration.

11. Procédé selon la revendication 9 ou la revendication 10,
**caractérisé en ce que**
le procédé comprend en outre une capture du flux d'images au moyen d'une caméra (4) et une visualisation du modèle dentaire (1) et de l'image bidimensionnelle (20) sur un dispositif d'affichage (12) pour un utilisateur, en particulier dans lequel le visage est le visage de l'utilisateur.

12. Procédé selon l'une quelconque des revendications 9 à 11,
**caractérisé en ce que**
la caméra (4) et le dispositif d'affichage (12) font partie du même dispositif mobile (10), dans lequel le procédé est effectué par l'intermédiaire d'un ou plusieurs algorithmes installés sur une unité de calcul du dispositif mobile (10).

13. Procédé selon l'une quelconque des revendications précédentes,
**caractérisé en ce que**
le modèle dentaire virtuel (1) est dans un format de maillage polygonal et comprend une pluralité de sommets, en particulier dans lequel le calcul de l'éclairage du modèle dentaire comprend un calcul d'un ombrage, dans lequel le calcul de l'ombrage est effectué dans un dispositif d'ombrage de sommets et/ou localement par sommet ou par fragment.

14. Dispositif mobile (10) comprenant une caméra (4) et un dispositif d'affichage (12) qui sont agencés de sorte que des images (20) du visage d'un utilisateur puissent être capturées par la caméra (4) pendant que l'utilisateur regarde le dispositif d'affichage (12),
**caractérisé en ce que**
le dispositif comprend une unité de calcul avec au moins un algorithme qui est adapté pour exécuter le procédé selon l'une des revendications précédentes.

15. Produit de programme informatique comprenant un code de programme qui est stocké sur un support lisible par machine et présentant des instructions exécutées par ordinateur pour mettre en œuvre le procédé selon l'une quelconque des revendications 1 à 13.
